# FASCICULE DE BREVET EUROPEEN

(11) **EP 3 713 883 B1**
(45) Date de publication et mention de la délivrance du brevet: **06.10.2021**
(21) Numéro de dépôt: 18792926.0
(22) Date de dépôt: 23.10.2018
(51) Int. Cl.: C02F 3/08

(54) **SYSTEME POUR LE TRAITEMENT BIOLOGIQUE DES EAUX**
SYSTEM ZUR BIOLOGISCHEN BEHANDLUNG VON WASSER
SYSTEM FOR THE BIOLOGICAL TREATMENT OF WATER

(30) Priorité: 23.11.2017 FR 1761119
(43) Date de publication de la demande: 30.09.2020
(73) Titulaire: Veolia Water Solutions & Technologies Support, 94417 Saint-Maurice Cedex (FR)
(72) Inventeur: SAUVIGNET, Philippe, 35460 Saint Etienne en Cogles (FR); MEUDAL, Nicolas, 6005 Luzern (CH); HERTFLEDER, Nikolai, 74592 Kirchberg And Des Jagst (DE); INIAL, Goulven, 6846 XB Arnhem (NL); LINDEGAARD, Sophie, 94100 Saint Maur Des Fosses (FR)
(74) Mandataire: Vidon Brevets & Stratégie
(86) Numéro de dépôt international: PCT/EP2018/078951
(87) Numéro de publication internationale: WO 2019/101454

(56) Documents cités:
- WO-A1-86/05770
- FR-A- 784 706
- GB-A- 508 881

## Description

### 1. Domaine de l'invention

Le domaine de l'invention est celui du traitement biologique des eaux, notamment en vue de leur épuration ou de leur potabilisation.

Plus précisément l'invention concerne un système de traitement biologique des eaux par biomasse fixée sur médias.

### 2. Art antérieur

Il existe plusieurs types de traitements biologiques d'eaux usées utilisant une biomasse fixée sur média. Contrairement aux boues activées dans lequel la biomasse peut se développer librement dans les eaux à traiter, la biomasse fixée sur médias colonise des médias introduits dans l'eau à traiter afin de former un film biologique ou biofilm. La fixation de la biomasse en biofilm permet de diminuer la taille du ou des réacteurs biologiques.

Il est notamment connu de l'art antérieur un procédé à « réacteur à film biologique à lit mobile» (en anglais: « *moving bed biofilm reactor* »), généralement dénommé par son acronyme anglais : MBBR. Un réacteur MBBR conventionnel consiste en un réacteur rempli de médias libres maintenus en mouvement par fluidisation. Une grille ayant un maillage plus petit que la taille des médias libres est disposée en sortie de réacteur afin de maintenir ces derniers à l'intérieur du réacteur. Le réacteur MBBR permet notamment le traitement du carbone et la nitrification des eaux usées par injection d'une grande quantité d'air. Cette injection d'air permet l'aération des eaux pour obtenir un traitement biologique aérobie ainsi que la fluidisation du lit de médias. Le procédé MBBR permet de traiter une grande quantité d'eau du fait de l'importante surface disponible offerte par les médias libres et est à ce jour principalement utilisé dans des stations de traitement des eaux usées de grande taille. Cependant il présente certains désavantages : l'injection en continu d'air dans le réacteur rend le coût d'exploitation du procédé élevé ; en outre, le nettoyage et la maintenance des buses d'aération est ardue, ces dernières étant peu accessibles.

La demande FR784706A décrit un système pour l'épuration des eaux mettant en oeuvre des supports poreux accueillant une biomasse, coopérant avec des disques rotatifs formant tambours destinés à être partiellement immergés dans l'eau à traiter.

La demande WO86/05770 décrit un système dans lequel l'eau à traiter est alimentée par des buses à la périphérie d'un tambour de façon à faire tourner celui-ci à la façon d'une roue à aube. Un système similaire est présenté dans la demande GB508881A, dont un mode de réalisation inclut au sein du tambour des tubes perforés solidarisé à celui-ci. FR2169266 décrit un système similaire dans lequel le tambour rotatif est entièrement rempli par des médias libres.

Il est également connu de l'art antérieur le procédé à « contacteur biologique rotatif » (en anglais : « *rotating biological contacter* »), également dénommé par son acronyme anglais RBC. Un réacteur RBC conventionnel consiste en une série de disques montés en parallèle sur un arbre central horizontal et placés dans un réservoir contenant une eau à traiter de manière à être partiellement immergés. Les disques sont généralement immergés à environ 40% de leur surface totale et mis en rotation autour de l'arbre central horizontal. Ainsi, les disques constituent un médium sur lequel un biofilm peut se développer. Au cours de la rotation des disques, le médium est alternativement submergé dans l'eau à traiter puis exposé à l'air ambiant. Ceci permet de réaliser un traitement biologique aérobie, notamment un traitement du carbone et une nitrification, sans injection d'air dans l'eau à traiter. Cependant, le procédé RBC présente plusieurs désavantages : Premièrement, la capacité d'oxygénation de la biomasse dans ce procédé est modérée et constitue un facteur limitant au développement de la biomasse. Deuxièmement, l'utilisation du médium n'est pas optimale puisque seulement une petite partie du médium (moins de 40%) est immergée et jamais la totalité. Troisièmement, la biomasse se développe de manière hétérogène sur la surface du médium : la biomasse se développe et s'accumule d'autant mieux au niveau des parties les plus proches de l'arbre horizontal du fait qu'elle est soumise à une plus faible vitesse radiale. Un déséquilibre de la répartition de la charge sur les disques implique l'utilisation de moteurs de forte puissance. Quatrièmement, le procédé RBC produit des quantités importantes par à coup de matières solides en suspension en sortie d'installation du fait du détachement d'une partie du biofilm. De manière plus générale, les contacteurs biologiques rotatifs permettent de traiter des volumes d'eau moindres du fait d'un encombrement relativement important de l'installation. De plus leur efficacité de traitement reste souvent aléatoire et peu prévisible.

### 3. Objectifs de l'invention

La présente invention a pour objectif de proposer un système de traitement biologique d'eaux par biomasse fixée sur médias qui pallie au moins certains des inconvénients de l'art antérieur cité ci-dessus.

Notamment, un objectif de l'invention est de fournir un système de traitement biologique d'eaux qui puisse traiter efficacement de grands volumes d'eaux à un coût de revient modéré.

Un autre objectif de l'invention est de fournir un système de traitement biologique d'eaux qui soit robuste et ne nécessitant que peu d'entretien.

Un autre objectif de l'invention, au moins selon certains modes de réalisation, est de fournir un système de traitement biologique d'eaux qui permette une bonne aération des eaux à traiter.

Un autre objectif est de proposer un procédé de traitement biologique d'eaux utilisant ledit système de traitement selon la présente invention.

### 4. Exposé de l'invention

Ces différents objectifs sont atteints, grâce à l'invention qui concerne un système pour le traitement biologique d'eaux usées comprenant :
- un réservoir comprenant un point d'entrée d'une eau à traiter et un point de sortie d'une eau traitée,
- un tambour rotatif définissant un volume interne, ledit tambour étant apte à tourner autour d'un axe horizontal et présentant une paroi au moins en partie perméable à l'eau, le tambour rotatif étant disposé dans le réservoir et étant destiné à être partiellement immergé dans les eaux contenues dans le réservoir, moins de la moitié de son volume interne baignant dans les eaux contenues dans le réservoir,
- un moyen d'entraînement destiné à entraîner le tambour rotatif en rotation autour de l'axe horizontal et,
- des médias libres, ayant une surface apte au développement d'un biofilm, disposés à l'intérieur du tambour rotatif et retenus par la paroi du tambour rotatif, les médias libres en vrac occupant un volume compris entre 30% et 80% du volume interne du tambour rotatif;
- au moins un tube creux s'étendant parallèlement audit axe horizontal fixé à ladite paroi dudit tambour rotatif à l'intérieur dudit tambour rotatif, ledit au moins un tube creux ayant une section longitudinale perforée comprenant un ensemble d'orifices répartis de manière homogène sur toute la longueur dudit au moins un tube creux, ledit au moins tube creux étant conçu pour se remplir alternativement d'eau et d'air lorsque ledit tambour rotatif, partiellement immergé dans les eaux contenues dans le réservoir, est mis en rotation dans un sens donné.

Les médias libre occupant 30 % à 80 % de ce volume, la rotation du tambour conduit au fait que ces médias sont tantôt émergés, tantôt immergés.

Ainsi, les inventeurs de la présente invention ont combiné de façon originale certaines des caractéristiques techniques des réacteurs MBBR, notamment l'utilisation de médias libres offrant ainsi une grande surface disponible de médias pour le développement d'un biofilm avec certaines caractéristiques des contacteurs biologiques rotatifs, notamment la mise en rotation des médias. Du fait de la mise en rotation du tambour rotatif, les médias peuvent être animés d'un mouvement continu à l'intérieur du tambour rotatif. Une croissance homogène de la biomasse sur les médias libres est possible grâce à un effet de chute. Par rapport à un réacteur MBBR qui nécessite une aération intense pour la fluidisation des médias libres, la mise en rotation des médias libres dans le tambour rotatif permet de s'approcher de conditions idéales d'un ensemble de médias libres infiniment mélangé dans un réacteur parfaitement agité, tout en s'affranchissant d'une aération dédiée. Par rapport à un contacteur biologique rotatif, la mise en rotation des médias libres dans le tambour rotatif permet d'assurer une croissance homogène sur les médias et un détachement progressif de biofilm mort, assurant ainsi une teneur en matières solides en suspension dans l'eau traitée ayant de faibles variations.

Le taux de remplissage en médias libres du tambour rotatif correspond au rapport du volume occupé par les médias libres en vrac par le volume intérieur du tambour rotatif. Un taux de remplissage en médias libres inférieur à 30% n'est pas souhaitable car cela engendrerait un encombrement excessif non justifié de l'installation au vu de ses capacités de traitement biologique et/ou une mauvaise efficacité du traitement biologique du fait de la faible surface offerte par les médias libres pour le développement d'un biofilm. Par ailleurs, un taux de remplissage en médias libres supérieur à 80 % n'est pas plus souhaitable . En effet, cela empêcherait le bon mélange des médias libres à l'intérieur du tambour rotatif, empêcherait le détachement du biofilm par frottement des médias entre eux et nuirait à la bonne aération du réacteur (sauf à prévoir une aération supplémentaire que celle autorisée par les tubes perforés).

Le réservoir utilisé peut être de même type que celui déjà utilisé dans les procédés RBC. En particulier, des contacteurs biologiques rotatifs peuvent facilement être convertis en système selon la présente invention par remplacement des disques par le tambour rotatif rempli de 30% à 80% en volume des médias libre. Le réservoir comprend un point d'entrée d'une eau à traiter et un point de sortie d'une eau traitée. Le point d'entrée de l'eau à traiter et le point de sortie de l'eau traitée sont avantageusement situés à deux extrémités opposées du réservoir. Le réservoir comprend optionnellement un déversoir placé à une altitude donnée du réservoir permettant de fixer un niveau maximum d'eau dans le réservoir.

Le tambour rotatif est apte à tourner autour d'un axe horizontal. Sa paroi est formée par un cylindre creux fermé par deux disques en ses extrémités. Elle est au moins en partie perméable à l'eau, c'est à dire qu'elle peut être traversée par l'eau contenue dans le réservoir au moins sur certaines sections. Elle est aussi apte à retenir l'ensemble des médias libres à l'intérieur du tambour rotatif. Le tambour rotatif est disposé dans le réservoir et est destiné à être partiellement immergé dans les eaux contenues dans le réservoir.

Avantageusement, la paroi en cylindre creux du tambour rotatif est formée d'un grillage ou d'une tôle perforée dont la maille ou taille des trous est suffisamment petite pour pouvoir retenir l'intégralité des médias libres à l'intérieur du tambour rotatif. Ceci permet notamment d'alléger le poids du tambour rotatif et d'assurer une bonne circulation d'eau entre l'intérieur et l'extérieur du tambour rotatif.

On notera que le tambour peut aussi être compartimenté au moins en deux parties pour assurer une meilleure rigidité du grillage ou de la tôle perforée.

Le tambour rotatif est apte à être mis en rotation autour de l'axe horizontal grâce à un moyen d'entrainement. Ce moyen d'entraînement comprend notamment un moteur et un arbre d'entraînement fixé sur ledit tambour rotatif selon l'axe horizontal.

Les médias libres sont disposés à l'intérieur du tambour rotatif et retenus par la paroi du tambour rotatif. En outre, ils occupent un volume compris entre 30% et 80% du volume interne du tambour rotatif. Cette fourchette de valeurs permet d'assurer une capacité de traitement biologique suffisante et en même temps un bon mélange des médias libres au sein du tambour rotatif tout en contribuant à une aération efficace.

Les médias libres ont une surface apte au développement d'un biofilm. Ils peuvent notamment être fabriqués dans un matériau appartenant au groupe constitué par le polyéthylène haute densité (PEHD), le polypropylène, avec ou sans additifs comme par exemple de la chaux.

Les médias libres ont une forme adaptée permettant le développement d'un biofilm sur une large surface de médias mais pour un petit volume de médias. Ceci a notamment pour avantage de disposer d'une surface de médias jusqu'à quatre fois supérieure à la surface d'un contacteur biologique rotatif de même encombrement. La surface des médias libres peut notamment être divisée en une surface externe, confrontée aux forces d'abrasion liées aux chocs des médias libres entre eux, et une surface protégée (surface complémentaire de la surface externe). La surface spécifique des médias libres est alors définie comme étant le rapport de la surface protégée d'un média libre sur le volume du média libre et s'exprime en m²/m³. Les médias libres ont préférentiellement une surface spécifique supérieure à 200 m²/m³, avantageusement comprise entre 200 et 1300 m²/m³.

Selon un mode de réalisation particulier, les médias libres sont des médias en matière plastique aptes à être utilisés dans un procédé MBBR, comme par exemple des médias commercialisés sous les dénominations commerciales K1, K3, K5, F3 par la société AnoxKaldnes. Il peuvent aussi être constitués de médias d'origine naturelle organique ou minérale tels que : morceaux de ponce, billes en argile expansée, granulats de boues, grains de charbon actif, billes de polystyrène, morceaux de liège, etc.

Selon une caractéristique préférentielle de l'invention, le système selon l'invention comprend des moyens d'aération des eaux contenues dans le réservoir. Ceci permet de mettre en œuvre des traitements biologiques en conditions aérobie.

Selon l'invention, les moyens d'aération comprennent au moins un tube creux s'étendant parallèlement à l'axe horizontal et fixé à la paroi du tambour rotatif à l'intérieur du tambour rotatif. Le tube creux présente des orifices sur toute sa longueur. Ces orifices sont d'une taille inférieure à celle des médias libres de façon telle que ces médias libres ne puissent pas rentrer à l'intérieur dudit tube creux.

Lorsque le tambour rotatif, partiellement immergé dans les eaux contenues dans le réservoir, est mis en rotation dans un sens donné, le tube creux perforé se remplit alternativement d'eau et d'air. Plus précisément, lorsque le tube creux perforé se trouve émergé, il se vide d'eau et se remplit d'air. A contrario, quand le tube creux perforé se trouve immergé il se vide d'air et se remplit d'eau. Ceci permet donc une aération des eaux à l'intérieur du tambour à bas coût en utilisant avantageusement l'énergie du moyen d'entrainement. En outre, un tel moyen d'aération n'implique que des opérations de maintenance très simples comparé à la maintenance devant être faite sur les buses d'aérations de réacteurs MBBR conventionnels. De manière préférentielle, le système ne comprend pas d'autre moyen d'aération.

Le nombre d'orifices, la taille des orifices et la surface totale et la répartition des orifices peuvent être adaptés en fonction de l'aération souhaitée et du type de media. Le « taux de perçage » correspondant à la surface du tube occupée par les orifices par rapport à la surface extérieure totale du tube est préférentiellement compris entre 2 et 6 %.

Les tubes pourront notamment être de section transversale circulaire ou carrée. Les orifices pourront quant à eux notamment être de forme circulaire ou oblongue.

Préférentiellement, la section longitudinale perforée du tube creux comprend un ensemble d'orifices répartis de manière homogène sur toute la longueur du tube creux.

De manière avantageuse, la section longitudinale perforée est orientée radialement de telle sorte que le tube creux soit apte à se vider d'air au moment où il est le plus immergé sous les eaux contenues dans le réservoir. Ceci assure une meilleure efficacité de l'aération. Une telle orientation radiale peut notamment être obtenue en plaçant les orifices du tube creux perforé vers l'avant dans le sens de rotation du tambour rotatif, éventuellement tournés de quelques degrés vers l'axe horizontal.

Préférentiellement, le système comprend au moins deux tubes creux perforés identiques. Les au moins deux tubes creux sont avantageusement disposés dans le tambour rotatif de manière diamétralement opposée ou le cas échéant selon une configuration en polygone régulier. Ceci permet d'obtenir une répartition homogène des charges dans le tambour rotatif. Avantageusement le système comprend de quatre à dix tubes creux perforés identiques.

Selon une autre caractéristique préférentielle de l'invention, le système comprend des moyens d'étanchéité à l'air extérieur, pouvant en pratique être distincts des moyens d'étanchéité à l'eau. Ceci permet de mettre en oeuvre des traitements biologiques en conditions anoxiques.

Selon un mode de réalisation particulier de l'invention, ces moyens d'étanchéité comprennent un carter surmontant le réservoir et le point d'entrée d'une eau à traiter du réservoir et le point de sortie d'une eau traitée du réservoir sont des siphons à liquide, rendant ainsi le réservoir complètement étanche à l'air. Ces moyens peuvent aussi inclure des moyens d'étanchéité à l'air prévu aux extrémités de l'axe du tambour, au niveau de son raccordement au réservoir.

La présente invention concerne également un procédé pour le traitement biologique d'eaux usées dans un système tel que précédemment décrit. Ce procédé comprend :
- une alimentation du réservoir en une eau à traiter,
- une mise en rotation du tambour rotatif, partiellement immergé dans les eaux contenues dans le réservoir et,
- une évacuation du réservoir en une eau traitée,

Préférentiellement, la vitesse de rotation dudit tambour est comprise entre 1 et 5 tours par minutes.

### 5. Liste des figures

L'invention, ainsi que les différents avantages qu'elle présente, seront plus facilement compris grâce à la description qui va suivre d'un mode non limitatif de réalisation d'un système et d'un procédé selon celle-ci, donnée en référence aux dessins, dans lesquels :
la figure 1 est une vue de dessus d'un mode de réalisation d'un système selon l'invention ;
la figure 2 est une vue de face, en coupe, du réservoir et du tambour rotatif du système selon la figure 1 ;
la figure 3 représente un média libre commercialisé sous la dénomination K3 par la société AnoxKaldnes ;
la figure 4 représente partiellement un mode de réalisation de tube creux perforé utilisé dans le cadre de ce mode de réalisation.

### 6. Description détaillée d'un mode de réalisation d'un système selon l'invention

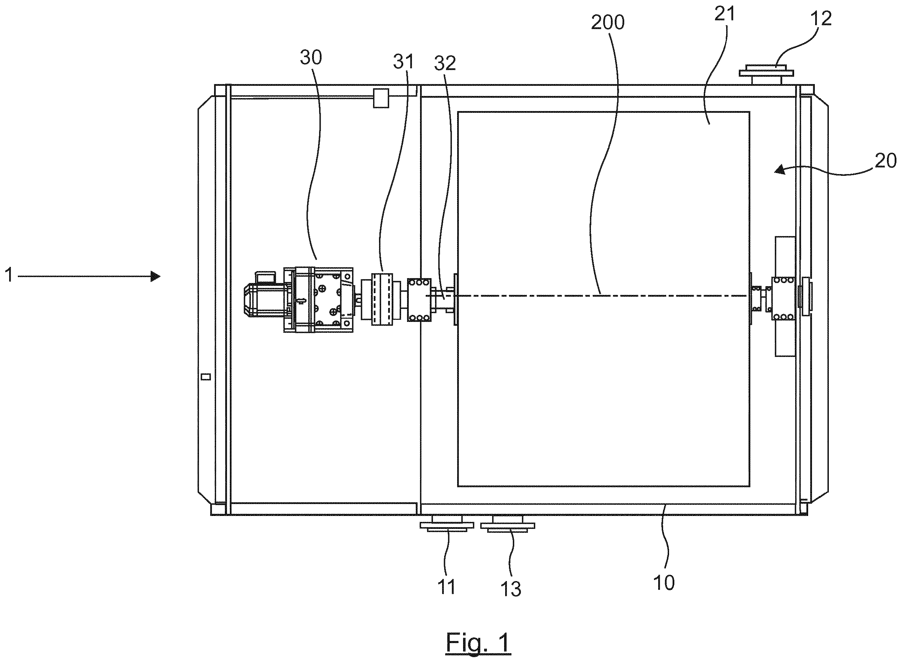

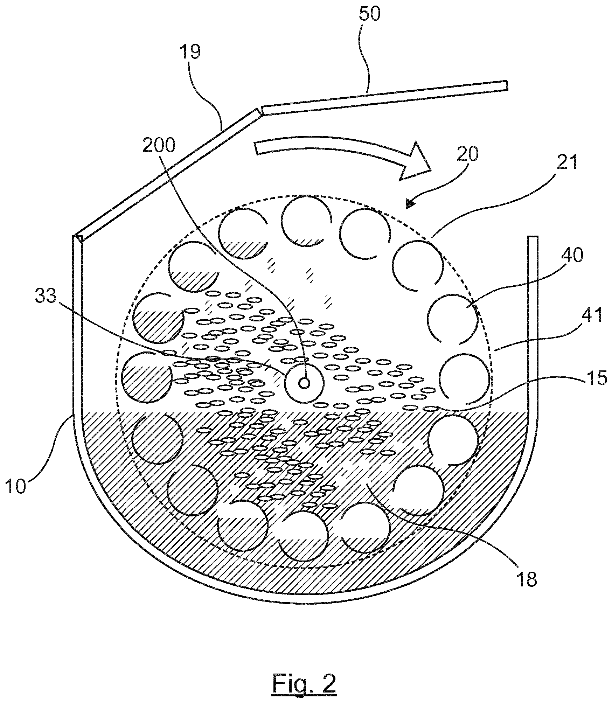

En référence aux Figures 1 et 2, le système comprend : un réservoir 10, un tambour rotatif 20, un moyen d'entrainement 30, 31, 32 permettant d'entrainer le tambour rotatif 20 en rotation, des médias libres 15 disposés à l'intérieur du tambour rotatif 20 et un moyen d'aération constitué de 10 tubes creux 40 perforés.

Le réservoir 10 est de forme hémicylindrique et comprend un point d'entrée 11 d'une eau à traiter et un point de sortie 12 d'une eau traitée. Le réservoir comprend également un déversoir 13 permettant de ne pas dépasser une hauteur maximale d'eau dans le réservoir.

Le tambour rotatif 20 peut tourner autour d'un axe horizontal 200 grâce à un moyen d'entrainement formé d'un motoréducteur 30, d'un arbre de transmission 32 et d'un accouplement élastique 31 entre le motoréducteur 30 et l'arbre de transmission 32. Le motoréducteur 30 peut être un motoréducteur de faible puissance, comme par exemple un motoréducteur de 1,5 kW.

Le tambour rotatif 20 a une paroi 21 constituée d'un cylindre creux fermé par deux disques à ses extrémités. La paroi en cylindre creux du tambour rotatif 20 est formée d'un grillage ou d'une tôle perforée dont le maillage est suffisamment petit pour pouvoir retenir l'intégralité des médias libres 15 à l'intérieur du tambour rotatif 20. La taille des mailles du tambour est dans ce mode de réalisation de 15 mm.

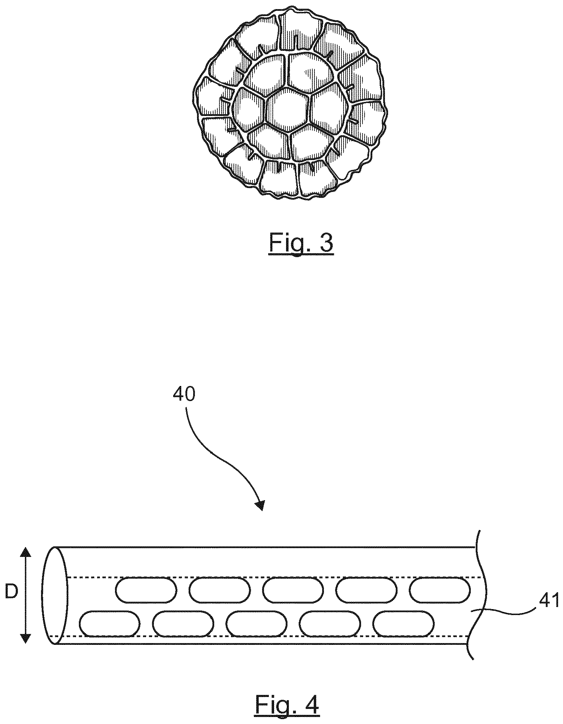

Les disques aux extrémités du cylindre creux sont fixés à l'arbre de transmission 32. Le tambour rotatif 20 est donc particulièrement léger. En référence à la Figure 4, chacun des dix tubes creux perforés 40 présente un diamètre D de 110 mm et est pourvu d'orifices répartis en deux lignes et placés en quinconce. Plus précisément, chaque tube présente quatre-vingt quatre orifices de 2 cm de diamètre, les orifices étant séparés les uns des autres par un entraxe de 3,5 cm environ. Cette répartition des orifices correspond à un taux de perçage des tubes de 6%.

Des médias libres 15, sont disposés à l'intérieur dudit tambour rotatif 20 et retenus par ladite paroi du tambour rotatif 20. Ils occupent un volume correspondant à 67 % du volume interne du tambour rotatif 20 soit environ 3,4 m³. Cette valeur permet d'assurer une capacité de traitement biologique suffisante et en même temps un bon mélange des médias libres au sein du tambour rotatif.

En référence à la Figure 3, représentant un média libre commercialisé sous la dénomination commerciale K3 par la société AnoxKaldnes, les médias libres utilisés ont une forme adaptée permettant le développement d'un biofilm sur une large surface de médias mais pour un petit volume de médias. Ces éléments présentent un diamètre de 25 mm, une hauteur de 10 mm et une surface protégée de 500 m2/m3.

Un traitement de type aérobie nécessite une bonne aération des eaux contenues dans le tambour rotatif 20. Cette aération est assurée par les tubes perforés 40 fixés à l'intérieur de la paroi 21 du tambour rotatif 20, parallèlement à l'axe horizontal 200. Les tubes creux 40 ayant une section longitudinale perforée 41, lorsque le tambour rotatif 20 tourne, les tubes creux 40 se remplissent alternativement d'eau et d'air, relarguant de l'air 18 dans les eaux contenues dans le tambour rotatif 20, les médias tombant en cascade à la surface des eaux contenues dans le tambour rotatif 20. Ainsi, l'aération et le mélange du tambour rotatif 20 sont générés par le même motoréducteur 30 et ne nécessitent que peu d'énergie.

Le nombre de perforations ainsi que la surface occupée par ces perforations est optimisée pour assurer un transfert d'air adéquat dans les eaux contenues dans le tambour rotatif 20.

L'orientation des tubes perforés 40 est optimisée afin que les tubes creux se vident d'air au moment où ils sont les plus immergés sous les eaux contenues dans le tambour rotatif 20.

Dans l'hypothèse où l'installation ici décrite serait utilisée pour un traitement en milieu anoxique, le carter 50 qui surmonte le réservoir 10 et le passage de l'axe 200 au travers du réservoir sont rendus complètement étanche à l'air.

### 7. Description d'un mode de réalisation d'un procédé selon l'invention

Le système décrit ci-dessus a été utilisé pour traiter des eaux présentant une DBO (demande biologique en oxygène) de 540 mg/L. On a utilisé un débit d'eau dans le système de 1 m³/h correspondant à un temps de rétention de l'eau dans le tambour d'environ 2,5 heures. Dans cet exemple, la vitesse de rotation du tambour a été fixée à 3,6 tours par minute ce qui a permis d'amener environ 31 m ³ d'air par heure dans l'eau présente dans le tambour, soit 6,3 m ³ d'air par heure et par mètre cube de tambour, correspondant à 0,019 m³ d'air apporté par heure et par mètre carré de surface de médias.

## Revendications

1. Système (1) pour le traitement biologique d'eaux usées comprenant :
- un réservoir (10) comprenant un point d'entrée (11) d'une eau à traiter et un point de sortie (12) d'une eau traitée,
- un tambour rotatif (20) définissant un volume interne, ledit tambour étant apte à tourner autour d'un axe horizontal (200) et présentant une paroi (21) au moins en partie perméable à l'eau, ledit tambour rotatif (20) étant disposé dans ledit réservoir (10) et étant destiné à être partiellement immergé dans les eaux contenues dans ledit réservoir (10), moins de la moitié de son volume interne baignant dans les eaux contenues dans le réservoir (10),
- un moyen d'entraînement (30, 31, 32) destiné à entraîner ledit tambour rotatif (20) en rotation selon ledit axe horizontal (200),
- des médias libres (15), ayant une surface apte au développement d'un biofilm, disposés à l'intérieur dudit tambour rotatif (20) et retenus par ladite paroi (21) dudit tambour rotatif (20), le taux de remplissage en médias libres du tambour rotatif (20) étant compris entre 30% et 80% du volume interne dudit tambour rotatif (20), où ledit taux de remplissage correspond au rapport du volume occupé par les médias libres en vrac par le volume intérieur du tambour rotatif (20);
- au moins un tube creux (40) s'étendant parallèlement audit axe horizontal (200) fixé à ladite paroi (21) dudit tambour rotatif (20) à l'intérieur dudit tambour rotatif (20), ledit au moins un tube creux (40) ayant une section longitudinale perforée comprenant un ensemble d'orifices répartis de manière homogène sur toute la longueur dudit au moins un tube creux (40), ledit au moins un tube creux étant conçus pour se remplir alternativement d'eau et d'air lorsque ledit tambour rotatif, partiellement immergé dans les eaux contenues dans le réservoir, est mis en rotation dans un sens donné.

2. Système (1) pour le traitement biologique d'eaux usées selon la revendication 1 **caractérisé en ce que** lesdits médias libres (15) ont une surface spécifique supérieure à 200 m2/m3.

3. Système (1) pour le traitement biologique d'eaux usées selon la revendication 2, **caractérisé en ce que** lesdits médias libres (15) ont une surface spécifique entre 200 et 1300 m²/m³.

4. Système (1) pour le traitement biologique d'eaux usées selon l'une des revendications 1 à 3 **caractérisé en ce qu'**il comprend au moins deux tubes creux (40) identiques, préférentiellement de quatre à dix tubes creux (40) identiques.

5. Système (1) pour le traitement biologique d'eaux usées selon l'une quelconque des revendications précédentes **caractérisé en ce qu'**il comprend des moyens d'étanchéité (50) à l'air extérieur.

6. Système (1) pour le traitement biologique d'eaux usées selon l'une quelconque des revendications précédentes caractérisé ce que lesdits médias libres sont en matirèe plastique.

7. Système (1) pour le traitement biologique d'eaux usées selon l'une quelconque des revendications 1 à 5 caractérisé ce que lesdits médias libres sont choisis dans le groupe constitué par les morceaux de pierre ponce, les billes en argile expansée, les granulats de boues, les grains de charbon actif, les billes de polystyrène, les morceaux de liège.

## Patentansprüche

1. System (1) zur biologischen Behandlung von Abwasser, umfassend:
einen Tank (10), umfassend eine Eintrittsstelle (11) für ein zu behandelndes Wasser und eine Austrittsstelle (12) für ein behandeltes Wasser,
- eine Drehtrommel (20), die ein Innenvolumen definiert, wobei die Trommel dazu geeignet ist, sich um eine horizontale Achse (200) zu drehen, und eine Wand (21) aufweist, die zumindest zum Teil wasserdurchlässig ist, wobei die Drehtrommel (20) in dem Tank (10) angeordnet ist und dazu vorgesehen ist, zum Teil in das in dem Tank (10) enthaltene Wasser versenkt zu werden, wobei weniger als die Hälfte seines Innenvolumens in das in dem Tank (10) enthaltene Wasser eingetaucht wird,
- ein Antriebsmittel (30, 31, 32), das dazu vorgesehen ist, die Drehtrommel (20) entlang der horizontalen Achse (200) drehend anzutreiben,
- freie Medien (15), die eine Oberfläche aufweisen, die zur Entwicklung eines Biofilms geeignet ist, im Inneren der Drehtrommel (20) angeordnet sind und durch die Wand (21) der Drehtrommel (20) zurückgehalten werden, wobei der Grad der Befüllung der Drehtrommel (20) mit freien Medien zwischen 30 % und 80 % des Innenvolumens der Drehtrommel (20) liegt, wobei der Grad der Befüllung dem Verhältnis des Volumens, das von den freien Medien als Schüttgut belegt wird, zu dem Innenvolumen der Drehtrommel (20) entspricht;
- mindestens eine hohle Röhre (40), die sich parallel zu der horizontalen Achse (200) erstreckt und von der Wand (21) der Drehtrommel (20) zum Inneren der Drehtrommel (20) befestigt ist, wobei die mindestens eine hohle Röhre (40) einen perforierten Längsabschnitt aufweist, der eine Menge von Öffnungen umfasst, die einheitlich auf der gesamten Länge der mindestens einen hohlen Röhre (40) verteilt sind, wobei die mindestens eine hohle Röhre dazu entworfen ist, sich abwechselnd mit Waser und mit Luft zu füllen, wenn die Drehtrommel, die zum Teil in das in dem Tank enthaltene Wasser versenkt ist, in einer gegebenen Richtung in Drehung versetzt wird.

2. System (1) zur biologischen Behandlung von Abwasser nach Anspruch 1, **dadurch gekennzeichnet, dass** die freien Medien (15) eine spezifische Oberfläche von mehr als 200 m²/m³ aufweisen.

3. System (1) zur biologischen Behandlung von Abwasser nach Anspruch 2, **dadurch gekennzeichnet, dass** die freien Medien (15) eine spezifische Oberfläche zwischen 200 und 1300 m²/m³ aufweisen.

4. System (1) zur biologischen Behandlung von Abwasser nach einem der Ansprüche 1 bis 3, **dadurch gekennzeichnet, dass** es mindestens zwei identische hohle Röhren (40), vorzugsweise vier bis zehn identische hohle Röhren (40) umfasst.

5. System (1) zur biologischen Behandlung von Abwasser nach einem der vorhergehenden Ansprüche, **dadurch gekennzeichnet, dass** es Mittel (50) zur Abdichtung gegen die Außenluft umfasst.

6. System (1) zur biologischen Behandlung von Abwasser nach einem der vorhergehenden Ansprüche, **dadurch gekennzeichnet, dass** die freien Medien aus Kunststoff sind.

7. System (1) zur biologischen Behandlung von Abwasser nach einem der Ansprüche 1 bis 5, **dadurch gekennzeichnet, dass** die freien Medien aus der Gruppe bestehend aus Bimssteinbrocken, Blähtonkügelchen, Schlammgranulat, Aktivkohlekörnern, Polystyrolkügelchen, Korkstücken ausgewählt sind.

## Claims

1. System (1) for the biological treatment of wastewater comprising:
- a tank (10) comprising a point for the entry (11) of water to be treated and a point for the exit (12) of treated water,
- a rotating drum (20) defining an internal volume, said drum being capable of rotating about a horizontal shaft (200) and having a wall (21) that is at least partly permeable to water, said rotating drum (20) being disposed in said tank (10) and being intended for being partially submerged in the water contained in said tank (10), less than half of its internal volume bathing in the water contained in the tank (10),
- a driving means (30, 31, 32) intended for driving said rotating drum (20) in rotation about said horizontal shaft (200),
- free media (15), having a surface suited to the development of a biofilm, disposed within said rotating drum (20) and retained by said wall (21) of said rotating drum (20), the fill level of free media (15) of said rotating drum (20) ranging from 30% to 80% of the internal volume of said rotating drum (20), wherein said fill level corresponds to the ratio of the volume occupied by the loose free media (15) to the internal volume of said rotating drum (20);
- at least one hollow tube (40) extending in parallel to said horizontal shaft (200) fixed to said wall (21) of said rotating drum (20) within said rotating drum (20), said at least one hollow tube having a perforated longitudinal section comprising a set of orifices distributed homogeneously throughout the length of said at least one hollow tube (40), said at least one hollow tube being designed to get filled alternately with water and with air when said rotating drum, partially submerged in the water contained in the tank, is put into rotation in a given sense.

2. System (1) for the biological treatment of wastewater according to claim 1 **characterized in that** said free media (15) have a specific surface area greater than 200 m²/m³.

3. System (1) for the biological treatment of wastewater according to claim 2, **characterized in that** said free media (15) have a specific surface area of 200 to 1300 m²/m³.

4. System (1) for the biological treatment of wastewater according to any one of the claims 1 to 3, **characterized in that** it comprises at least two identical hollow tubes (40), preferably four to six identical hollow tubes (40).

5. System (1) for the biological treatment of wastewater according to any one of the above claims, **characterized in that** it comprises means (50) of airtightness against external air.

6. System (1) for the biological treatment of wastewater according to any one of the above claims, **characterized in that** said free media are made of plastic.

7. System (1) for the biological treatment of wastewater according to any one of the claims 1 to 5, **characterized in that** said free media are chosen from the group constituted by pieces of pumice stone, expanded clay beads, sludge granulates, granular activated carbon, polystyrene beads, pieces of cork.
